Europäisches Patentamt

European Patent Office

Office européen des brevets

(11) Publication number: 0 329 356
A2

# EUROPEAN PATENT APPLICATION

(21) Application number: 89301327.6

(51) Int. Cl.⁴: G06F 15/20

(22) Date of filing: 13.02.89

(30) Priority: 17.02.88 US 157324

(43) Date of publication of application:
23.08.89 Bulletin 89/34

(84) Designated Contracting States:
AT BE CH DE ES FR GB GR IT LI LU NL SE

(71) Applicant: WAYNE STATE UNIVERSITY
380 Mackenzie Hall
Detroit Michigan 48202(US)

(72) Inventor: Spitzer, Robert
4375 Boreland Avenue
West Bloomfield Michigan 48033(US)
Inventor: Hassoun, Mohamed
5650 Middlesex
Dearbon Michigan 48126(US)

(74) Representative: Wharton, Peter Robert
Urquhart-Dykes & Lord Alliance House 29-31
Kirkgate
Bradford West Yorkshire, BD1 1QB(GB)

(54) Physiologic waveform analysis.

(57) A waveform analysis assembly (10) includes a sensor (12) for detecting physiological electrical and mechanical signals produced by the body. An extraction neural network (22) will learn a repetitive waveform over a first pass of the electrical signal and store the waveform in memory (18). During a second pass over the electrical signal, the waveform is extracted from the electrical signal and times of occurrence of the waveform are also stored in a buffer (30). A third pass subtracts the waveform from the electrical signal for reprocessing finding an additional waveform. The process is repeated for each significantly different waveform in the electrical signal. A reducer (20) receives the stored waveforms and times and reduces them into features characterizing the waveforms. A classifier neural network (36) analyzes the features by classifying them with standard features representing diseased states and outputs results of diseased states based on the best match of the features to the normal and patient groups.

# PHYSIOLOGIC WAVEFORM ANALYSIS

## TECHNICAL FIELD

The invention relates to analyzing waveforms of physiological signals produced by the body for determining diseased states.

## BACKGROUND OF THE INVENTION

Electromyography (EMG) is an important tool in the diagnosis of diseases of the peripheral nervous system. An EMG signal is recorded from a needle inserted into a specified muscle, and represents the electrical discharge of groups of muscle fibers. Abnormality is estimated by observing the potentials on an oscilloscope screen. Reliability of the technique for the diagnosis of diseases of the peripheral nervous system have been seriously limited by a lack of a method to accurately and quickly qualify features of the EMG. Additionally, extension of the use of the EMG to diagnosis of disorders of the central nervous system has been limited by an ability to accurately measure pattern information by visual estimation. In visual diagnosis, the physician sees potentials that flicker across the oscilloscope screen at 10 to 30 Hz, and listens to the sound on a loud speaker. The results are also highly dependent on the training of the individual performing the examination and subject to bias. This accounts for limitations on the reproducibility and reliability of the test in diagnosis of diseases of the peripheral nervous system. Another significant limitation is the inability of the observer to quantify certain perimeters such as firing rate and pattern, and relationships between firing patterns and recruitment of units. While attempts have been made by more experienced physicians and researchers to extend EMG to the diagnosis of diseases of the central nervous system, an ability to accurately measure appropriate perimeters have prevented realization of this goal.

Previous attempts to apply computer analysis to the EMG signal have been marginally successful because the signal is extremely variable and complex. Recently, new methods based on modelling of processing (computation) by biologic neurons have demonstrated better capabilities in the traditional algorithm for analyzing complex signals such as images.

A first method for a motor unit quantitation was developed by Buchthal. "Action Potential Parameters in Normal Muscle and Their Dependence on Physical Variables", F. Buchthal, C. Gold, P.

Rosenfalck. Acta Physiol Scand, 1954a (32) 200. His method involves the recording of randomly collective motor units on photographic film or paper. The motor units are visually inspected, and duration, amplitude and phases measured and tabulated. After 20 or more units are measured, resulting mean values are compared with normative data collected by Buchthal. This method is extremely time consuming, taking up to an hour for a signal muscle. Since most clinical studies involve examining up to a dozen muscles or more, this method is not practical except in a research setting. Again, it is subject to significant biased by the individual selecting and measuring the units.

Several computer assisted methods of MUP quantization have been developed initially as research tools. The computer programs have generally been developed on laboratory mini computers, usually PDP-11, and after they have been published they have been made available as software packages.

One of the best efforts to date has been by Dorfman and McGill. "Automated Decomposition of the Clinical Electromyogram,," K.C. McGill, K.L. Cummins, L.J. Dorfman. IEEE Trans. Biomed. Eng., 32 (7): 470-477, July 1985. This program is called ADEMG (Automated Decomposition of the EMG). The program records the interference pattern at threshold, 10% or 30% of maximal effort. It then filters and differentiates the signal to locate the motor unit spikes. Motor units are isolated and compared by a templet matching scheme. Recurrences of the same unit are aligned in the Fourier domain, and averaged. Superimpositions are resolved whenever possible during matching. A final average is then reprocessed to remove adjacent or overlapping units that may be degrading the average. Duration is computed automatically. Firing rate is analyzed, but is used merely to locate missing occurrences of motor units. No further processing is performed. This method may erroneously resolves polyphasic motor units into discrete components, thus failing in disease states where motor unit phases are increased. While the program tracks slowly varying wave shape, it has trouble detecting repeated occurrences of the same when that unit is unstable in configuration, as is the case in several disease states. Under these circumstances, ADEMG may erroneously detect the slightly different occurrences as distinct motor units.

The most significant signal processing method has been by Gevins. "Igorance-based Neural-Network Signal Processing in Brain Research", Alan S. Gevins and Nelson H. Morgan, June 1987. The method outlined in the paper looks at the application of neural-network classifier-directed methods to neurological waveform detection. The methods include application to contaminant detection for replacement of ad-hoc detectors, and waveform detection for evoked potential estimation.

In the application of contaminants, expert knowledge of contaminant types is represented by training data which have been hand-marked, which is used to train a neural network to distinguish clean from contaminated data. Several neural networks are used, each trained to detect a different type of contaminant. The network of this method is incapable of receiving a large number of features and classifying the input on best match. Furthermore, the method does not disclose any type of initial waveform identification. In the evoked potential estimation, assumptions about the signal and noise properties are necessary based on potential. This method requires pre-conceived assumptions of the input signal in order to operate.

The prior art requires prior information regarding the data signal in order to process the waveforms. Problems arise when the waveform does not match any of the original assumptions, or there is a superimposition of waveforms.

## SUMMARY OF THE INVENTION

The invention is the method of discriminating physiological signals and a wave analysis assembly for discriminating physiological signals produced within the body and for producing output indicative of disease characteristics. The assembly includes sensing means for detecting physiological signals produced within the body and producing an electrical signal. The assembly also includes extraction network means for receiving the physiological signal for learning a repetitive waveform of the electrical signal and for extracting the learned waveform from the electrical signal. The extraction network means includes memory means for storing the extracted waveform. Output means outputs information of disease diagnosis based on the extracted waveform. The assembly also includes classifier means containing information comprising standard features and combinations thereof associated with disease states for comparing the features to the information to produce an output representative of diseased states based on the best match of the features with the information.

The assembly is advantageous over the prior art in that the assembly will learn a repetitive waveform and is therefore able to handle superimpositions, polyphasic waveforms, and non-templated waveforms. Additionally, the assembly also will produce the best match of diseased state without requiring an exact match of all features. In other words, the assembly may receive an unknown data signal and learn a repetitive waveform from that signal without any pre-conceived information regarding the waveform, and extract the learned waveform from the data signal.

## BRIEF DESCRIPTION OF THE DRAWINGS

Other advantages of the present invention will be readily appreciated as the same becomes better understood by reference to the following detailed description when considered in connection with the accompanying drawings wherein:

Figure 1 is a block diagram of the subject invention;

Figures 2a and b are the flow chart defining the block diagram of Figure 1;

Figure 3 illustrates a simulated electromyogram containing two motor units and noise;

Figure 4 illustrates a learned motor unit and a sampled motor unit for extraction;

Figure 5 illustrates reconstruction of a motor unit; and

Figure 6 illustrates the electrical signal after subtraction of the first MU.

## DESCRIPTION OF THE PREFERRED EMBODIMENT

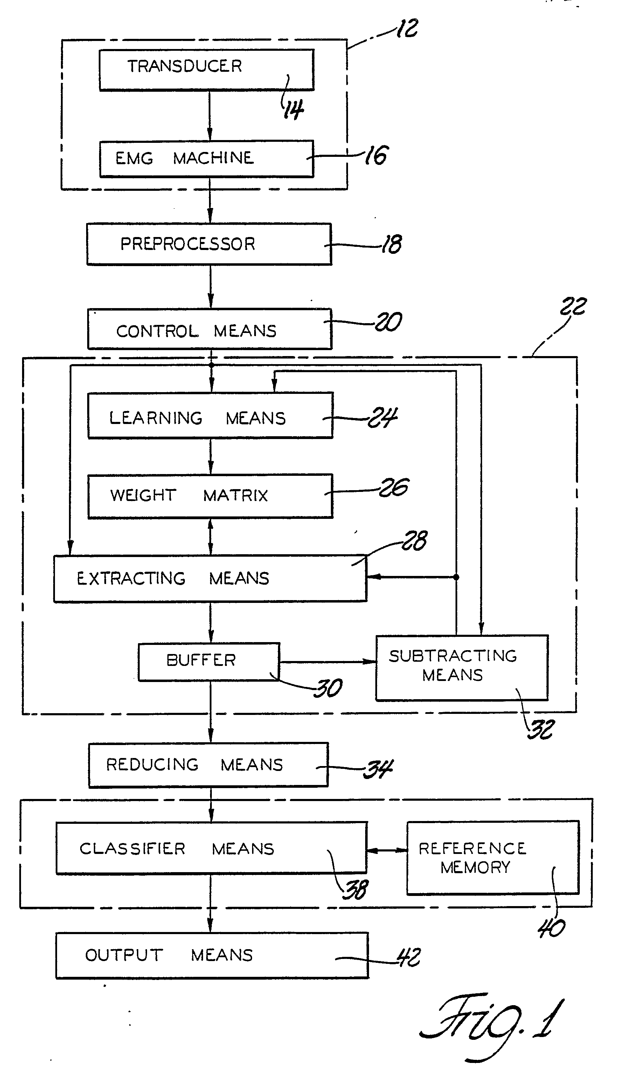

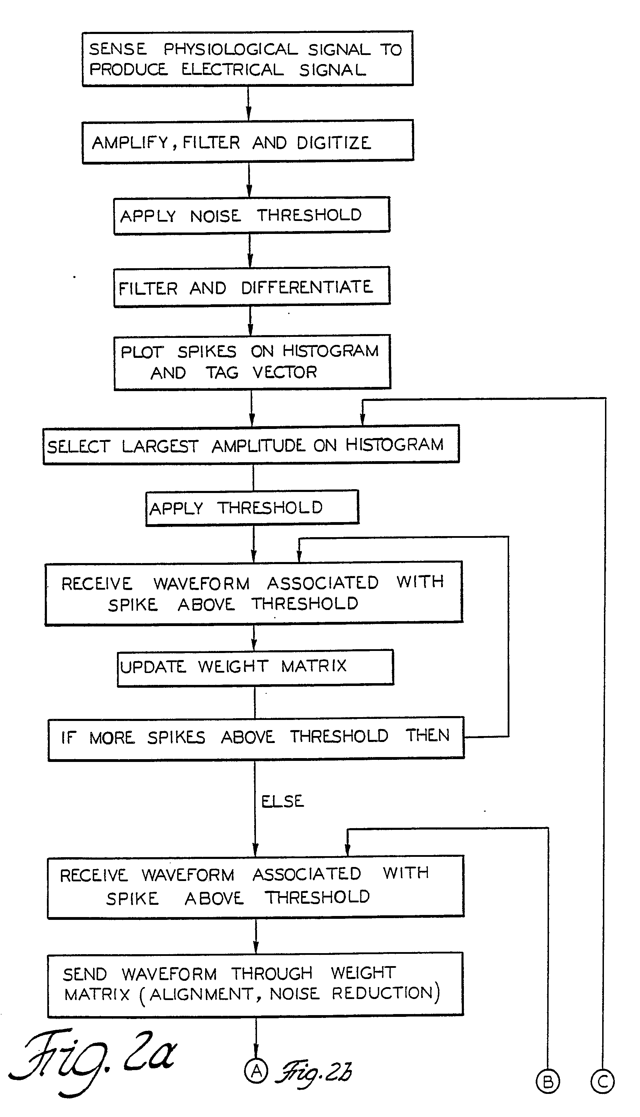

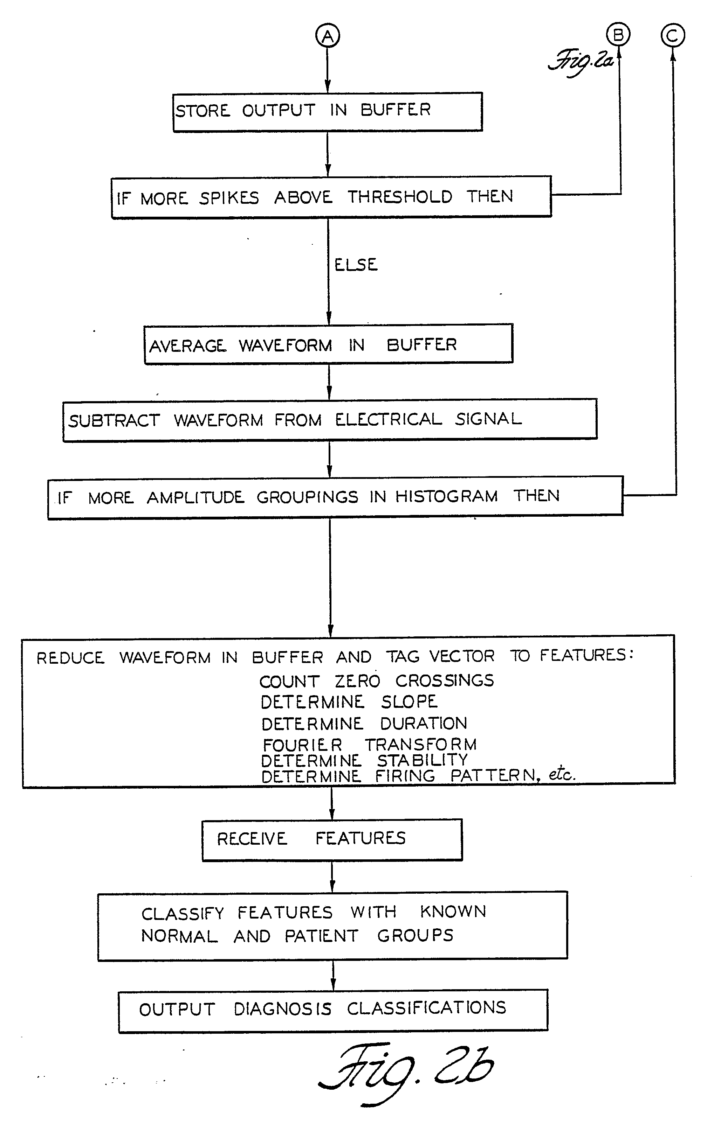

A wave analysis assembly is generally shown at 10 in Figure 1 which uses the flow chart in Figure 2. The wave analysis assembly and method may be applied to any physiological electrical or mechanical signals sensed from internal activity, such as electromyography (EMG), electroencephylogram (EEG), and electrocardiogram (EKG), etc. The preferred embodiment of the subject invention is directed toward diagnosing neuromuscular disorders and will be defined in this application, but the apparatus and method may be applied to detecting and analyzing physiologic waveforms from any source. Electromyography is a technique for diagnosing neuromuscular disorders by analyzing the electrical signal recorded from a contracting muscle using a needle electrode 12.

By way of background, the electrical signal recorded as an electromyogram (EMG), is made of trains of discrete waves called motor unit potentials. The motor unit potentials (MUP) may be defined as the electrical potential within a muscle produced by the simultaneous discharge of all the muscle fibers connected to a single motor neuron. The lower motor neuron is the neuron in the spinal cord who's axon directly connects to a group of muscle fibers in a specific muscle. Each discharge of this neuron directly discharges the muscle fibers, causing them to contract. The firing of this neuron is under the control of the other neurons in the central nervous system, including the upper motor neurons. Because of the intimate relationship with the peripheral nerve and muscle fibers, this neuron can properly be considered functionally part of the peripheral nervous system, even though it is physically within the central nervous system.

A neuron's major property is its ability to produce a frequency output in response to the processing of multiple inputs from other neurons. The same term is used to indicate a mathematically modeled neuron which is the basic element of a neural network.

A neutral network, also termed parallel distributed processing, perceptron, associative memory, etc., is a simulated or physically constructed array of modeled neurons. The array has input connections, interconnections between neurons, and output connections, generally called the synaptic weight matrix. The simplest arrays are linear, but more complex architectures are under investigation. The processing accomplished by the array occurs in parallel, and is dependent on both the physical architecture of interconnection, and the strengths of interconnection or the elements.

As indicated in Figures 1 and 2, the assembly 10 includes the general steps of sampling the physiological signal 12, preprocessing 18 the signal, learning and extracting 22 a waveform, reducing 34 the waveform to features, and classifying 36 the feature to produce a diagnostic information or interpretable output. A user interactive system will allow review of the raw electrical signal and extracted motor units to verify accurate performance. Each step will be subsequently described in detail.

Figure 1 illustrates the block diagram of the subject invention, and Figure 2 illustrates the associated flow chart for implementing the means of Figure 1.

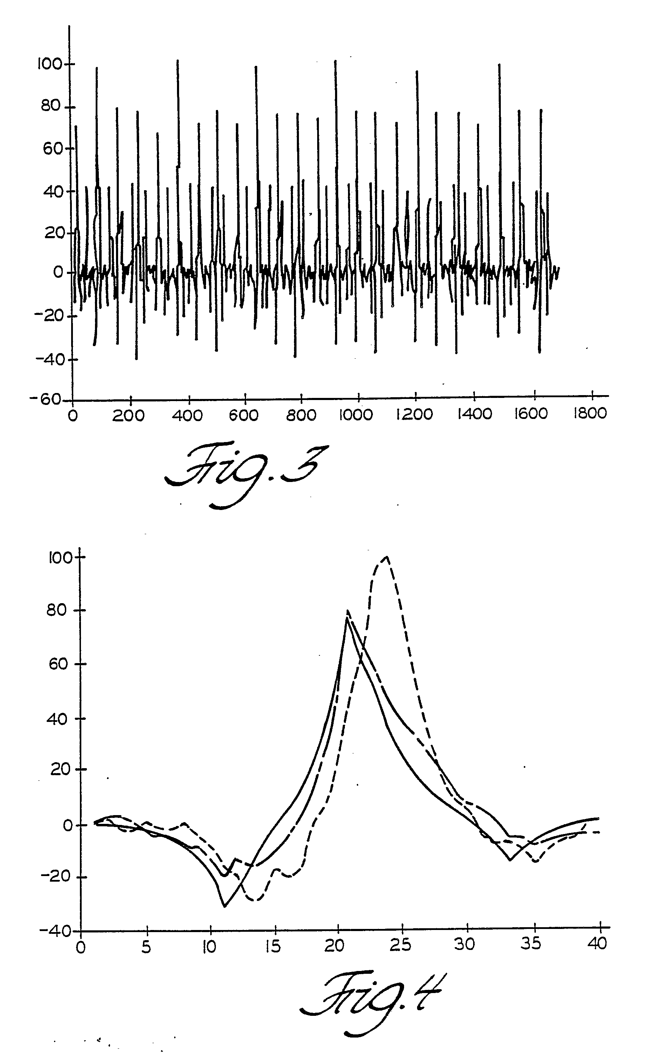

The assembly 10 includes sensing means 12 for sampling the physiological signal produced within the body and for producing an electrical or original signal. The sampling means 12 may include any type of transducer for converting the sensed data into an electrical signal. The sensing means 12 is includes a concentric needle electrode or monopolar needle electrode 14. The sensing means 12 also includes a standard EMG machine 16 which amplifies and filters the signal from the needle and an analog to digital converter producing a digitized electrical signal for processing. Figure 3 illustrates a simulated EMG signal as would be sensed and which includes the repetitive occurrence of two motor units. Preferably, ten seconds of EMG signal will be sampled at each needle 14 insertion site. Since typical motor unit firing rates are approximately 10 Hz, it is estimated that 100 occurrences of each unit will be obtained in the sampled sweep. The EMG will be analyzed at minimal or threshold effort and at 10% and 30% of maximal effort. Experience has indicated that up to five or six units can be analyzed at each site, but the invention is not limited to this number.

The assembly 10 also includes preprocessing means 18 for filtering and differentiating the electrical signal to isolate spikes associated with the waveforms. The preprocessing 18 may incorporate the method as set out in the McGill reference comprising filtering and identification of the motor unit spike locations. The noise level of the electrical signal is determined using the algorithm developed by McGill and Dorfman, this establishes a threshold below which peaks in the signal are not considered to represent candidate motor units. A standard upper filter setting of 10 KHz and a sample rate of 20KHz is preferably used. Initial work suggests Fourier interpolation will not be necessary, but this can be added later if needed. The spikes generally indicate the location of a motor unit occurrence, and its associated waveform. Peaks in the waveform are detected by a simple sign-change algorithm that approximates a differentiator and zero crossing detector.

The assembly 10 includes control means 20 for identifying spikes associated with individual waveforms and their respective locations. The control means 20 may be simply implemented through software. The spikes are sorted by amplitude, and an amplitude histogram is made and used to isolate the groups of amplitude. The amplitude of every spike is taken to make the histogram. A histogram is a plot of the amplitude versus the number of occurrences. A tag vector associated with all the detected peaks is also made. The tag rector indicates the time location of the peak occurrences. The spikes for a single motor unit tend to cluster around a single amplitude. It is assumed for the assembly 10 that each peak or grouping of potential represents one motor unit. The histogram has peaks and troughs. A threshold is selected for each trough in the amplitude histogram. Processing begins with the group of spikes associated with the amplitudes lying to the right of the first trough in the histogram. These spikes are identified by simple threshold detection, with the trough value determined from the histogram determining this threshold. The tag vector locations associated with these spikes are likely to identify the largest motor unit potential waveform in the data.

The assembly 10 includes extracting network means 22 for receiving the electrical signal and for learning the repetitive waveform components within the electrical signal and for extracting the learned waveform. The extracting network means 22 is a neural network. The configuration of a neural network is commonly known in the art and will not be described in detail herein. The extracting network means 22 includes buffer 30 means 30 for storing the extracted waveform and associated times of occurrence of the waveform within the electrical signal. Peaks less than a specified magnitude, for example 100uV, are assumed to be noise and are not processed. Therefore, only spikes and associated waveforms above the threshold will be learned and extracted.

The first step of the extracting neural network 22 is the learning step implemented by learning means 24. Each waveform associated with an isolated spike will be analyzed. The data centered at each detected spike above the highest threshold is fed into the network 22 from the control means 20. The neural network 22 will make a pass over the electrical signal and learn the appearance of each motor unit at each spike location in the histogram grouping. The learning means 24 can learn any number of linked waveforms, polyphasic waveforms, or complex waveforms but will reject superimpositions. An entire waveform, which may be 256 sample points, is received in parallel. Each occurrence of a waveform will be taken and fed into the neural network 22. Each motor unit identified in this manner is processed by the extracting neural network 22 separately, in reverse order with respect to amplitude. Therefore, the motor unit associated with the largest amplitude will be processed first.

The first step of the extraction network 22, which is performed by the learning means 24, is to make a first pass over the signal and synthesize a weight matrix for a motor unit. The extraction network 22 is controlled by the control means 20 as to which waveform to learn an respective locations of the spike associated therewith, by applying the threshold. It is to be understood, that the extraction network 22 may implement the functions of the control means 20, thus eliminating the necessity of the control means 20. The weight matrix 26 is the configuration used to describe the form of storage and processing of a motor unit waveform within a neural network 22, as is known in the art. The learning means 24 receives the sampled data points of a motor unit waveform and stores the information in the weight matrix 26 in a representative distributed fashion called the synaptic weight measurement. The array of inputs or sampled points surrounding a peak or spike are fed into the first neural network 22. The system generally looks at a 10-20 msec duration or window of a motor unit, but may obtain a maximum window of generally 100 msec, but this window may vary. Single motor units with waveforms greater than 100 msec would be obvious to a diagnosing physician of a disease. If there are two motor units within a window, the neural network 22 initially assumes a complex waveform. If this is in fact not a complex waveform but a superimposition, it is unlikely to occur often within the complete 10 second sampling period, and will therefore be "forgotten". Since a single motor unit is consecutively detected approximately 100 times within the 10 msec sampling period and learned, a few wrong or odd waves will not effect the learned waveform. If the two waves or superimposition do always occur, the learning means 24 of the neural network 22 can detect and learn this waveform. Therefore, the learning means 24 will search the electrical signal for each peak or waveform associated with the specific amplitude grouping of the histogram, and will learn the waveform by adjusting the waveform by a factor of 1/K upon detection of each spike of the specific amplitude. The learning means 24 will look at K waveforms, approximately 100 waveforms but which may vary, and learn the waveform by updating the learned waveform in an averaging fashion. In other words, the learning means 24 of the neural network 22 receives the first waveform approximately associated with the amplitude grouping from the histogram. The learning means 24 stores this waveform in the weight matrix 26 and then detects another waveform of similar amplitude and "averages" them by a factor of 1/k. This process is continued until the first pass is completed and all waveforms above the threshold are learned. Upon completion of this learning pass, an idealized representation of the motor unit potential waveform resides in the network's weight matrix 26 in a distributed fashion. Therefore, any repetitive waveform shape may be learned and stored in the weight matrix 26.

During the second pass of the electrical signal by the neural network 22, there is no further learning. The second pass is an extraction pass which implemented by extracting means 28 within the neural network means 22. The weight matrix 26 is not adjusted. The idealized representation of the motor unit waveform is now retrieved from the weight matrix 26 of the network 22. The same occurrences centered at the same spikes as during the learning pass are used as input. As each candidate occurrence of the motor unit is presented, the extracting means 28 responds with an output that represents an extraction of the underlying motor unit potential and is stored in a summing buffer 30. This extracted waveform retrieved by the extracting means 28 is aligned in the least-squares sense. The extraction pass includes aligning, and removing noise and superimpositions as indicated in Figure 4. If the amplitude or waveform shape is very different from the learned waveform, this will be indicated as wrong and ignored. Slight variations will not throw off the extracting means 28, and it will look for these occurrences. Therefore, the original electrical signal is received and compared to the learned waveform representation in the weight matrix 26, and the output which is aligned and noise removed is sent to a summing buffer 30 for averaging. A time table or tag rector in the buffer 30 is produced indicating the time of each occurrence of the motor unit. The firing pattern associated with this time table of a single motor unit is used to investigate possible intermediate occurrences of the motor unit that were missed by the amplitude criterion. Spikes in the vicinity of such missing locations are evaluated by the neural network 22 to determine if they represent the unit being analyzed. The firing pattern, thus extracted, represents an important feature of the signal, and is used for classification. When there is a match of the waveform in the electrical signal, the time occurrence is stored and the waveform is extracted from the electrical signal to the summing buffer 30. The differences are extracted and superimpositions that do not match are thrown out of the electrical signal output of the second pass. In other words, for each input, the resultant network output is summed in the buffer 30 and the final result averaged. Automatic alignment is accomplished during the extraction pass. Figure 6 indicates a resulting signal after the extraction of a motor unit from the original signal of Figure 3. The process of learning and extracting is repeated for each motor unit and associated waveform.

After one motor unit is learned, the third step of the network 22 is to subtract the motor unit from the electrical signal at each tag vector location before processing another motor unit which is implemented by subtracting means 32. Since the neural network learns the entire shape of the waveform within its window, including all adjoining peaks that belong to the same motor unit, this step removes all associated peaks. This includes all of the peaks associated with polyphasic or satellite waveforms. Complex waveforms are not processed incorrectly as separate motor unit potentials.

After the first and greatest amplitude grouping from the histogram is analyzed by this process and subtracted form the electrical signal, the next greatest amplitude grouping is analyzed which is presumed to be associated with a another motor unit. This process continues for each amplitude grouping of the histogram, and the learned and extracted waveform of each associated motor unit is stored in a separate buffer 30. It is to be noted that for subsequent motor units, the resultant signal outputted from the subtracting means 32 will be used as the electrical signal for the next learning and extraction. When another motor unit is to be processed, the previous already processed motor units have been subtracted from the electrical signal and are therefore not included in the processed signal, as indicated in Figure 6.

In other words, the neural network 22 detects and learns and the extracts one waveform, and then will repeat to find another waveform based on the next largest amplitude of the histogram. During the subtraction step, the learned waveform is aligned on the largest peak of the sampled waveform. The system can analyze polyphasic and subtract slight variations. Peaks less than 100uV are assumed to be noise and are not processed.

As an alternative embodiment of the extraction network 22, the neural network 22 may learn a repetitive waveform and during the third pass extract any unique or substantially different waveform from the electrical signal and store that unique waveform in the buffer 30 for reducing 34. This would be an ideal application of EKG signals wherein unique signals other than the repetitive signal may be detected and subtracted by the network 22. The learned waveform may be input by the user, instead of learning. The network 22 then may use this templated waveform for the extraction. Either similar or dissimilar waveforms may be extracted.

Generally, network 22 as implemented, may receive an electrical signal or data and learn any repetitive waveform with the signal. The same electrical signal is used for extracting a waveform based on the learned waveform. In other words, an unknown is used for learning, and the unknown data are also used for the extracting. No preconceived waveform is necessary. The source of the electrical signal is irrelevant for the network 22 as long as there is a repetitive waveform.

The assembly 10 includes reducing means 34 for analyzing the waveforms in the buffers 30 to produce features separately characterizing each waveform. Once the motor unit waveforms are obtained, there is a standard procedure to the reduction of the information into tabulated features. Such a procedure includes looking at zero crossings, the slope, duration and amplitude, etc. The reducing means 34 is implemented through software. The reducing means 34 will look at each waveform in the buffers 30 and the time table, along with the original sampled electrical signal. Examples of features obtained are mean duration, histograms of duration, amplitude, number of turns of the waveform, Fourier transform-median frequency or high and low frequency contents ratio, stability of waveform (compared with every occurrence in original data-extract variation), firing pattern (regular or irregular - and relation to amount of muscle force using and number of MUs used). This list is not exhaustive, and the more features used in characterizing the waveform, the better the classification, as subsequently described.

The assembly 10 includes classifier network means 36. The classifier network means 36 includes reference memory means 40 for containing information comprising standard features and combinations thereof associated with disease states for comparing the features to the information to produce an output representative of diseased states based on the best match of the features with the information. The classifier network means 36 comprises a different neural network processor. The features from the reducing means 34 are fed into the classifier neural network 36 and the features of the waveforms are compared to the information representing normal and patient groups characteristic features which represents the normal and disease states. This normal and patient groups information is stored in the reference memory means 24 of the classifier neural network 36 in weight matrix form and is characterized by the same type of features as those characterizing the motor unit waveforms from the reducing means 34. The features of the motor unit waveforms are compared to this information and the best matches are noted. All the features are considered in determining the pattern of features and an output is given to categorize them. The classifier neural network 36 takes the total number of features and classifies them giving the best match. Wrong or deviant features may be ignored in obtaining the best category.

The classifier neural network 36 includes an output means 42 for outputting physiologic information to aid the physician in diagnosing the disease. The physiologic output categories or classification may include disease output such as muscle disease, upper motor neuron disease, axonal neuropathy, neuro muscular junction disease, etc. The neural network 36 will output percentage ratings to the different physiologic diseases from 0% to 100% such that the physician will be informed of multiple symptoms and the proportional percentage of likelihood. In addition to the physiologic outputs, the original electrical signal and extracted motor units are printed out for consideration by the physician. The output means 42 may include a printer or graphic device for printing out hard copies of all the information recited above.

The invention includes a method of discriminating physiological electrical signals produced within the body and producing output indicative of disease characteristics. The method includes the steps of sensing 12 the electrical signals produced within the body, applying a noise threshold, filtering and differentiating 18 the electrical signal to isolate spikes associated with occurrences of the repetitive waveform, identifying spikes and plotting the histogram, applying the trough threshold to identify spikes associated with a single motor unit, learning 24 a repetitive waveform within the electrical signal by updating the weight matrix 26, extracting 28 the learned waveform from the electrical signal, storing 30 the extracted waveform and averaging the waveforms associated with a singles motor unit and storing 30 associated times of occurrence of the waveform within the electrical signal, subtracting the extracted waveform from the electrical signal, reducing 34 the waveform into features characterizing the waveform, analyzing 36 the features with respect to information comprising standard features and combinations thereof associated with disease states, and outputting 42 diseased states data based on the best match of the features with the information.

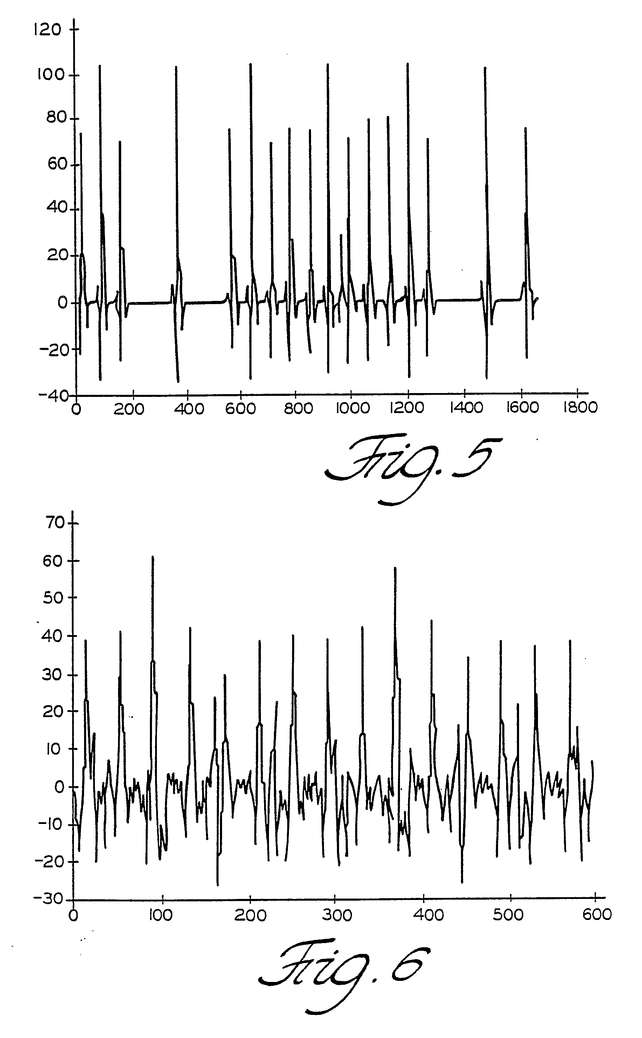

Figures 3 through 6 illustrate the effects of the subject invention as applied to an electrical signal. Figure 3 illustrates a simulated EMG signal comprised of two motor units with regular firing rates superimposed and with noise added. The EMG signal is detected by the sensing means 12 and then sent through the preprocessing means 18 for filtering and differentiation. A histogram will be made by the extraction network means 22, and a first motor unit grouping selected representing the greatest amplitude grouping. The motor unit is learned. Figure 4 indicates the learned motor unit waveform (solid line), and a misaligned, noisy occurrence (large dashes) presented to the extraction network 22, and the output (dotted line) is aligned and noise is rejected. Figure 5 indicates reconstruction of one motor unit by the extraction network 22. Firing information may then easily be derived and the missing occurrences checked (gaps on time line). The output in Figure 5 may be printed out for the physicians review, but otherwise the information is stored in the timetable and buffer 30 30. Figure 6 indicates the electrical signal after the first motor unit is subtracted. The remaining peaks correspond to the second motor unit. The process is then repeated to learn and extract the second motor unit. The learned waveform of Figure 4 is the waveform stored in the buffer 30 for the first motor unit, which is then reduced by the reducing means 34.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims wherein reference numerals are merely for convenience and are not to be in any way limiting, the invention may be practiced otherwise than as specifically described.

**Claims**

1. A wave analysis assembly for discriminating physiological signals produced within the body and for producing output indicative of disease characteristics, said assembly comprising;
sensing means (12) for sampling a physiological signal produced within the body and for producing an electrical signal, extraction network means (22) for receiving said electrical signal and for learning repetitive waveform components within said electrical signal and for extracting said learned waveform from said electrical signal, and output means (42) for outputting information of disease diagnosis based on said extracted waveform.

2. An assembly as set forth in claim 1 further characterized by including preprocessing means (18) for filtering and differentiating said electrical signal to isolate spikes associated with waveforms.

3. An assembly as set forth in claim 2 further characterized by including control means (20) for identifying the location of waveforms within said electrical signal.

4. An assembly as set forth in claim 1 further characterized by said sensing means (12) including a concentric needle.

5. An assembly as set forth in claim 1 further characterized by including reducing means (34) for analyzing said waveform to produce features characterizing said waveform.

6. An assembly as set forth in claim 5 further characterized by including classifier network means (36) containing information comprising standard features and combinations thereof associated with disease states for comparing said features to said information to produce an output representative of diseased states based on the best match of said features with said information.

7. An assembly as set forth in claim 1 further characterized by said extraction network means (22) including buffer means (30) for storing said extracted waveform within said electrical signal.

8. An assembly as set forth in claim 1 further characterized by said extraction network means (22) including learning means (24) for learning said repetitive waveform and a weight matrix (26) for storing said learned waveform.

9. An assembly as set forth in claim 1 further characterized by said extraction network means (22) including subtracting means (32) for subtracting said extracted waveform from said electrical signal.

10. A wave analysis assembly for discriminating physiological signals produced within the body and for producing output indicative of disease characteristics and including means for producing features characterizing the physiological signal, said assembly comprising; a classifier neural network (36) containing information comprising known features and combinations thereof associated with disease and normal patient states for comparing the features characterizing the physiological signal in relation to said information to produce an output representative of diseased states based on the best match of the features with said information.

11. An assembly as set forth in claim 10 further characterized by including sensing means (12) for sampling a physiological signal produced within the body and for producing an electrical signal.

12. An assembly as set forth in claim 10 further characterized by including reducing means (36) for analyzing said electrical signal and for producing features characterizing said electrical signal.

13. An assembly as set forth in claim 11 further characterized by including extraction network means (22) for receiving said electrical signal to identify and learn a repetitive waveform comprising said electrical signal and for extracting said learned waveform, said extraction network means (22) including buffer means (30) for storing said extracted waveform and associated times of occurrence of substantially similar waveforms within said electrical signal.

14. An assembly as set forth in claim 11 further characterized by including preprocessing means (18) for filtering and differentiating said electrical signal to isolate spikes associated with waveforms.

15. An assembly as set forth in claim 13 further characterized by including control means (20) for controlling said extraction network means (22) and for identifying waveforms in said electrical signal.

16. A method of discriminating physiological signals produced within the body and producing output indicative of disease characteristics, said method comprising the steps of: sensing the physiological signals produced within the body and producing an electrical signal, learning a repetitive waveform within the electrical signal, extracting the learned waveform from the electrical signal, and outputting information of the disease diagnosis based on the extracted waveform.

17. A method as set forth in claim 16 further including filtering and differentiating the electrical signal to isolate spikes associated with occurrences of the repetitive waveform.

18. A method as set forth in claim 16 further including locating the occurrences of waveforms within the electrical signal.

19. A method as set forth in claim 16 further including reducing the waveform into features characterizing the waveform.

20. A method as set forth in claim 19 further including analyzing the features with respect to information comprising standard features and combinations thereof associated with disease states.

21. A method as set forth in claim 20 further including outputting diseased states data based on the best match of the features with the information.

22. A method as set forth in claim 16 further including storing the extracted waveform and associated times of occurrence of the waveform within the electrical signal.

23. A method as set forth in claim 16 further including subtracting the extracted waveform from the electrical signal.

24. A wave analysis assembly for discriminating physiological signals produced within the body and for producing output indicative of disease characteristics, said assembly comprising; sensing means (12) for sampling a physiological signal produced within the body and for producing an electrical signal, an extraction neural network (22) for learning a waveform and for extracting a waveform from the electrical signal based on the learned waveform, and output means (42) for outputting information of disease diagnosis based on said extracted waveform.

25. An assembly as set forth in claim 24 further characterized by said extraction neural network (16) including learning means (22) for learning a repetitive waveform in electrical signal for the extraction.

26. An assembly as set forth in claim 24 further characterized by said extraction neural network (16) including learning means (22) for learning an template waveform inputted by the user for the extraction.

27. An assembly as set forth in claim 24 further characterized by said extraction neural network (16) including extracting means (28) for extracting waveforms from said electrical signal substantially similar to said learned waveform.

28. An assembly as set forth in claim 24 further characterized by said extraction neural network (16) . including extracting means (28) for extracting waveforms from said electrical signal substantially different from said learned waveform.

29. A method of discriminating physiological signals produced within the body and producing output indicative of disease characteristics, the method including the steps of;
sensing a physiological signal produced within the body and producing an electrical signal, learning a waveform, extracting a waveform from the electrical signal based on the learned waveform, and outputting information of the extracted waveform.

30. A wave analysis assembly for discriminating electrical signals, said assembly comprising; sensing means for inputting an electrical signal, extraction network means for receiving said electrical signal and for learning repetitive waveform components within said electrical signal and for extracting said learned waveform from said electrical signal, and output means for outputting information of said extracted waveform.

31. A method of discriminating electrical signals, said method including the steps of; inputting an electrical signal, learning a repetitive waveform within the electrical signal, extracting the learned waveform from the electrical signal, and outputting information based on the extracted waveform.

*Fig. 1*

Fig. 2a    Ⓐ Fig. 2b

Ⓐ     Ⓑ   Ⓒ

*Fig. 2a*

STORE OUTPUT IN BUFFER

IF MORE SPIKES ABOVE THRESHOLD THEN

ELSE

AVERAGE WAVEFORM IN BUFFER

SUBTRACT WAVEFORM FROM ELECTRICAL SIGNAL

IF MORE AMPLITUDE GROUPINGS IN HISTOGRAM THEN

REDUCE WAVEFORM IN BUFFER AND TAG VECTOR TO FEATURES:
COUNT ZERO CROSSINGS
DETERMINE SLOPE
DETERMINE DURATION
FOURIER TRANSFORM
DETERMINE STABILITY
DETERMINE FIRING PATTERN, *etc.*

RECEIVE FEATURES

CLASSIFY FEATURES WITH KNOWN
NORMAL AND PATIENT GROUPS

OUTPUT DIAGNOSIS CLASSIFICATIONS

*Fig. 2b*

Fig.3

Fig.4

*Fig. 5*

*Fig. 6*